# EUROPEAN PATENT SPECIFICATION

(11) **EP 3 856 883 B1**
(45) Date of publication and mention of the grant of the patent: **05.06.2024**
(21) Application number: 19866322.1
(22) Date of filing: 26.09.2019
(51) Int. Cl.: C11D 1/72, G01N 33/52, C11D 3/04, C11D 3/20

(54) **WASH REAGENT CONTAINING ALKOXYLATED FATTY ALCOHOL**
WASCHREAGENZ MIT ALKOXYLIERTEM FETTALKOHOL
RÉACTIF DE LAVAGE CONTENANT DE L'ALCOOL GRAS ALCOXYLÉ

(30) Priority: 28.09.2018 US 201862739150 P
(43) Date of publication of application: 04.08.2021
(73) Proprietor: Siemens Healthcare Diagnostics, Inc., Tarrytown, NY 10591 (US)
(72) Inventor: WANG, Yuan, Mountain Lakes, New Jersey 07046 (US); LANGLEY, Robert, Mauricetown, New Jersey 08329 (US); MARINO, Robert, Oakland, New Jersey 07436 (US); GINLEY, Patrick, New York 10990 (US)
(74) Representative: Siemens Healthineers Patent Attorneys
(86) International application number: PCT/US2019/053147
(87) International publication number: WO 2020/069124

(56) References cited:
- EP-A1- 3 015 538
- US-A- 5 888 752
- US-A- 5 888 752
- US-A1- 2003 215 890
- US-A1- 2003 215 890
- US-A1- 2018 002 634
- US-A1- 2018 265 808
- US-B2- 8 623 804
- ANONYMOUS: "ADVIA 2120 /2120I HEMATOLOGY SYSTEMS OPERATOR'S GUIDE", ADVIA 2120 /2120I HEMATOLOGY SYSTEMS, 1 March 2008 (2008-03-01), XP055835705,

## Description

### BACKGROUND

Automated or semi-automated hematology systems are well known in the art and include (but are not limited to) the ADVIA^{®} 120, ADVIA^{®} 2120, and ADVIA^{®} 2120i Hematology Systems (Siemens Healthcare Diagnostics, inc., Tarrytown, NY). These hematology systems streamline workflow by eliminating the majority of manual steps commonly performed to maximize productivity. However, the high-volume handling of these hematology systems results in buildup in various portions of the systems, such as (but not limited to) the Perox Vacuum Shuttle Chamber (VSC) and surrounding pathways, thus resulting in the clogging of the Unified Fluid Circuit (UFC) of the system. As the UFC is integrally formed, blockage in any area thereof requires replacement of the entire UFC.

In the past, a number of reagents and methods have been tested for the ability to remove buildup in hematology analyzers. These reagents/methods include the use of caustic reagents such as warm/highly concentrated sodium hydroxide solution as well as organic solvents such as (but not limited to) DMSO, ethanol, isopropanol, and benzol. Though some of these reagents demonstrated the ability to remove the Perox buildups, a sufficiently high concentration level was required for this ability to be seen, and these reagents are not compatible with the UFC material (which is typically acrylic) at such high concentrations. A hematology system which is a fully automated diagnostic instrument with a throughput of 120 samples per hour is disclosed in ANONYMOUS: ADVIA 21/20/2120I HEMATOLOGY SYSTEMS OPERATOR's GUIDE", March 2008. Further, document US 5 888 752 A is directed to the analysis of whole blood samples using automated homology systems and a reagent composition employed therein. This document discloses the use of a rinse solution comprising non-ionic surfactant.

Thus, a wash reagent has not yet been identified that is capable of removing the Perox buildups without damaging the UFC unit in hematology systems that contain Perox Vacuum Shuttle Chambers. Therefore, clogging of the UFC unit typically leads to a premature replacement of the entire UFC unit of the hematology system, which can be quite costly.

Therefore, there is a need in the art for new and improved wash reagents and methods of using same to minimize the Perox buildups in a hematology system without damaging the system. It is to such reagents, as well as methods of producing and using same, that the present disclosure is directed.

### BRIEF DESCRIPTION OF THE DRAWINGS

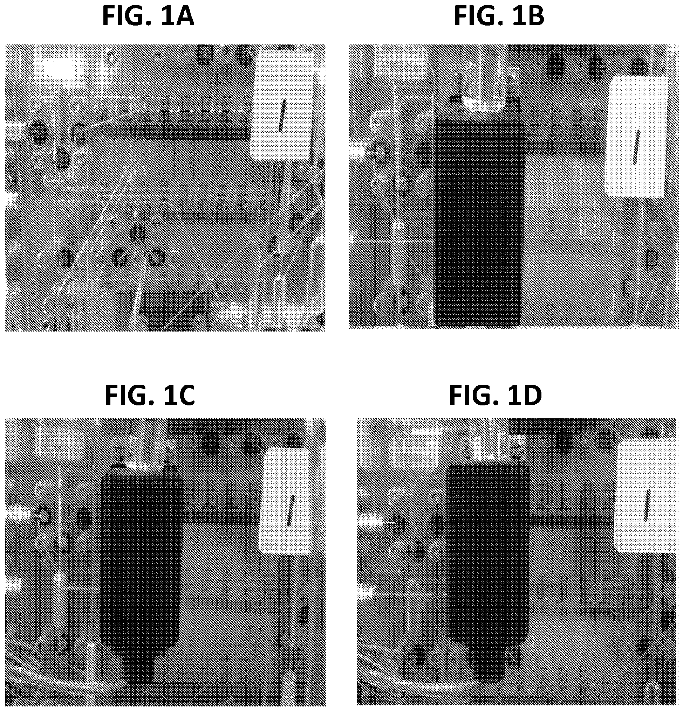

FIGS. 1A-1D contain photographs illustrating the cleaning of a first UFC Block of an ADVIA^{®} analyzer (designated therein by the number "1") with a wash reagent produced in accordance with the present disclosure. FIG. 1A: UFC Block #1 prior to beginning the cleaning process. FIGS. 1B and 1C: UFC Block #1 during two stages of the cleaning process. FIG. 1D: UFC Block #1 after the cleaning process.

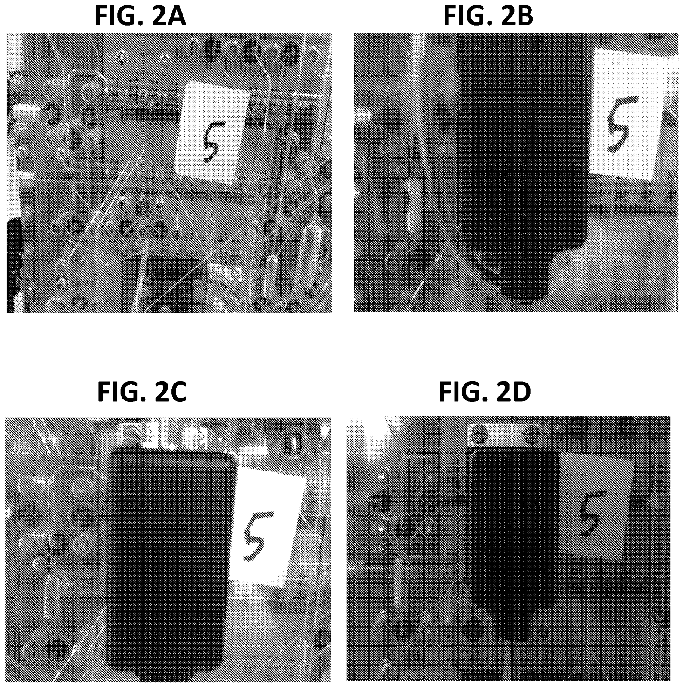

FIGS. 2A-2D contain photographs illustrating the cleaning of a second UFC Block (designated therein by the number "5") with a wash reagent produced in accordance with the present disclosure. FIG. 2A: UFC Block #5 prior to beginning the cleaning process. FIGS. 2B and 2C: UFC Block #5 during two stages of the cleaning process. FIG. 2D: UFC Block #5 after the cleaning process.

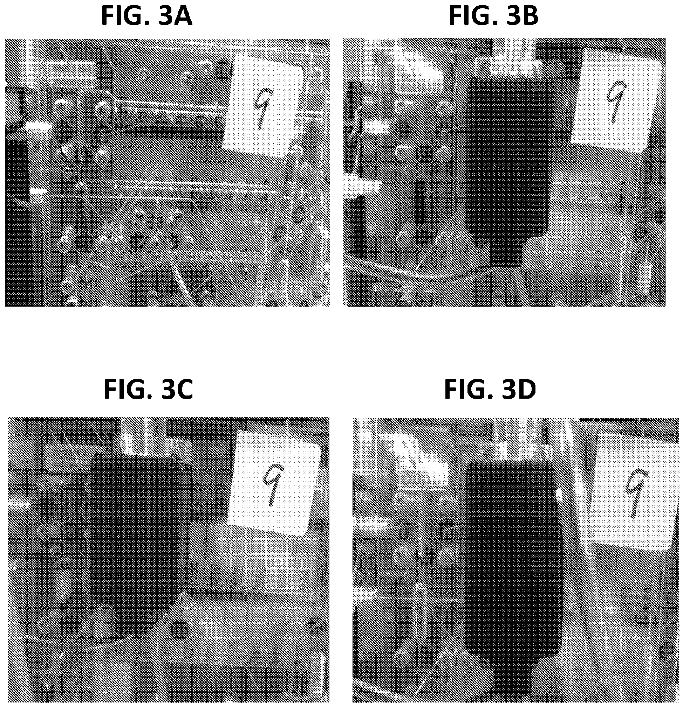

FIGS. 3A-3D contain photographs illustrating the cleaning of a third UFC Block (designated therein by the number "9") with a wash reagent produced in accordance with the present disclosure. FIG. 3A: UFC Block #9 prior to beginning the cleaning process. FIGS. 3B and 3C: UFC Block #9 during two stages of the cleaning process. FIG. 3D: UFC Block #9 after the cleaning process.

### DETAILED DESCRIPTION

Before explaining at least one embodiment of the present disclosure in detail by way of exemplary language and results, it is to be understood that the present disclosure is not limited in its application to the details of construction and the arrangement of the components set forth in the following description. The present disclosure is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the medical procedures and techniques of, surgery, anesthesia, wound healing, and infectious control described herein are those well-known and commonly used in the art. Standard techniques are used for infection diagnostic and therapeutic applications.

All patents, published patent applications, and non-patent publications mentioned in the specification are indicative of the level of skill of those skilled in the art to which the present disclosure pertains.

As utilized in accordance with the present disclosure, the following terms, unless otherwise indicated, shall be understood to have the following meanings:

The use of the term "a" or "an" when used in conjunction with the term "comprising" in the claims and/or the specification may mean "one," but it is also consistent with the meaning of "one or more," "at least one," and "one or more than one." As such, the terms "a," "an," and "the" include plural referents unless the context clearly indicates otherwise. Thus, for example, reference to "a compound" may refer to one or more compounds, two or more compounds, three or more compounds, four or more compounds, or greater numbers of compounds. The term "plurality" refers to "two or more."

The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z.

The use of ordinal number terminology (i.e., "first," "second," "third," "fourth," etc.) is solely for the purpose of differentiating between two or more items and, unless explicitly stated otherwise, is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used herein, any reference to "one embodiment," "an embodiment," "some embodiments," "one example," "for example," or "an example" means that a particular element, feature, structure, or characteristic described in connection with the embodiment is included in at least one embodiment. The appearance of the phrase "in some embodiments" or "one example" in various places in the specification is not necessarily all referring to the same embodiment, for example. Further, all references to one or more embodiments or examples are to be construed as non-limiting to the claims.

Throughout this application, the term "about" is used to indicate that a value includes the inherent variation of error for a composition/apparatus/ device, the method being employed to determine the value, or the variation that exists among the study subjects. For example, but not by way of limitation, when the term "about" is utilized, the designated value may vary by plus or minus twenty percent, or fifteen percent, or twelve percent, or eleven percent, or ten percent, or nine percent, or eight percent, or seven percent, or six percent, or five percent, or four percent, or three percent, or two percent, or one percent from the specified value, as such variations are appropriate to perform the disclosed methods and as understood by persons having ordinary skill in the art.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps. For example, a process, method, article, or apparatus that comprises a list of elements is not necessarily limited to only those elements but may include other elements not expressly listed or inherently present therein.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, when associated with a particular event or circumstance, the term "substantially" means that the subsequently described event or circumstance occurs at least 80% of the time, or at least 85% of the time, or at least 90% of the time, or at least 95% of the time. The term "substantially adjacent" may mean that two items are 100% adjacent to one another, or that the two items are within close proximity to one another but not 100% adjacent to one another, or that a portion of one of the two items is not 100% adjacent to the other item but is within close proximity to the other item.

As used herein, the phrases "associated with" and "coupled to" include both direct association/binding of two moieties to one another as well as indirect association/binding of two moieties to one another. Non-limiting examples of associations/couplings include covalent binding of one moiety to another moiety either by a direct bond or through a spacer group, non-covalent binding of one moiety to another moiety either directly or by means of specific binding pair members bound to the moieties, incorporation of one moiety into another moiety such as by dissolving one moiety in another moiety or by synthesis, and coating one moiety on another moiety, for example.

The present disclosure is directed to a kit according to claim 10.

The present disclosure is directed to a wash reagent according to claim 1.

In certain particular non-limiting embodiments, the alkoxylated fatty alcohol group comprises at least three degrees of ethoxylation. For example (but not by way of limitation), the alkoxylated fatty alcohol group comprises three degrees, four degrees, five degrees, six degrees, seven degrees, eight degrees, nine degrees, 10 degrees, 11 degrees, 12 degrees, 13 degrees, 14 degrees, 15 degrees, 16 degrees, 17 degrees, 18 degrees, 19 degrees, 20 degrees, or more degrees of ethoxylation, as well as any range between two of these values (such as (but not limited to) a range of from about three degrees to about 20 degrees, a range of from about five degrees to about 15 degrees, etc.).

In certain particular non-limiting embodiments, the number of carbons present in the primary alcohol chain of the alkoxylated fatty alcohol group is in a range of from about five to about 46 carbons. For example (but not by way of limitation), the number of carbons present in the primary alcohol chain of the alkoxylated fatty alcohol group may be about five, about six, about seven, about eight, about nine, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, and about 46 carbons, as well as any range between two of these values (such as (but not limited to) a range of from about 10 to about 40 carbons, a range of from about 13 to about 35 carbons, etc.).

In a particular (but non-limiting) embodiment, the alkoxylated fatty alcohol group comprises at least three degrees of ethoxylation, and the number of carbons present in the primary alcohol chain of the alkoxylated fatty alcohol group is in a range of from about five to about 46 carbons.

One particular (but non-limiting) example of a non-ionic surfactant with an alkoxylated fatty alcohol group that can be used in accordance with the present disclosure is NATSURF^{™} 265 (Croda Europe Limited, Snaith, United Kingdom).

Sodium hydroxide may be present at any amount that is sufficient to provide the wash reagent with an alkaline pH and that will allow the wash reagent to function in accordance with the present disclosure (i.e., the wash reagent should be capable of cleaning at least a portion of a hematology analyzer without substantially damaging the analyzer). For example (but not by way of limitation), sodium hydroxide may be present in an amount sufficient to provide the wash reagent with a pH in a range of from about 10 to about 14, such as (but not limited to), a pH of about 10, about 10.25, about 10.5, about 10.75, about 11, about 11.1, about 11.2, about 11.3, about 11.4, about 11.5, about 11.6, about 11.7, about 11.8, about 11.9, about 12, about 12.1, about 12.2, about 12.3, about 12.4, about 12.5, about 12.6, about 12.7, about 12.8, about 12.9, about 13, about 13.1, about 13.2, about 13.3, about 13.4, about 13.5, about 13.6, about 13.7, about 13.8, about 13.9, and about 14, as well as any range in between two of the above values (such as (but not limited to) a range of from about 11.7 to about 13.2, a range of from about 12.1 to about 12.9, etc.).

Each of the components of the wash reagent may be present at any concentration, so long as the wash reagent is capable of functioning in accordance with the present disclosure (i.e., the wash reagent should be capable of cleaning at least a portion of a hematology analyzer without substantially damaging the analyzer). For example (but not by way of limitation), the combination of sodium hydroxide, lauryl dimethyl amine oxide, 2-(2-ethoxyethoxy)ethanol, and processed water may present at a combined concentration in a range of from about 50 wt% to about 95 wt%. In particular (but non-limiting) examples, the combination of these four reagents may be present at a combined concentration of about 50 wt%, about 51 wt%, about 52 wt%, about 53 wt%, about 54 wt%, about 55 wt%, about 56 wt%, about 57 wt%, about 58 wt%, about 59 wt%, about 60 wt%, about 61 wt%, about 62 wt%, about 63 wt%, about 64 wt%, about 65 wt%, about 66 wt%, about 67 wt%, about 68 wt%, about 69 wt%, about 70 wt%, about 71 wt%, about 72 wt%, about 73 wt%, about 74 wt%, about 75 wt%, about 76 wt%, about 77 wt%, about 78 wt%, about 79 wt%, about 80 wt%, about 81 wt%, about 82 wt%, about 83 wt%, about 84 wt%, about 85 wt%, about 86 wt%, about 87 wt%, about 88 wt%, about 89 wt%, about 90 wt%, about 91 wt%, about 92 wt%, about 93 wt%, about 94 wt%, and 95 wt%, as well as any range in between two of the above values (such as (but not limited to) a range of from about 60 wt% to about 90 wt%, a range of from about 70 wt% to about 85 wt%, etc.).

In addition, the at least one non-ionic surfactant with the alkoxylated fatty alcohol group may be present at any concentration, so long as the wash reagent containing the non-ionic surfactant is capable of functioning in accordance with the present disclosure (i.e., the wash reagent should be capable of cleaning at least a portion of a hematology analyzer without substantially damaging the analyzer). For example (but not by way of limitation), the non-ionic surfactant with the alkoxylated fatty alcohol group may be present at a concentration in a range of from about 5 wt% to about 50 wt%. In particular (but non-limiting) examples, the non-ionic surfactant with the alkoxylated fatty alcohol group may be present at a concentration of about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, about 20 wt%, about 21 wt%, about 22 wt%, about 23 wt%, about 24 wt%, about 25 wt%, about 25 wt%, about 27 wt%, about 28 wt%, about 29 wt%, about 30 wt%, about 31 wt%, about 32 wt%, about 33 wt%, about 34 wt%, about 35 wt%, about 36 wt%, about 37 wt%, about 38 wt%, about 39 wt%, about 40 wt%, about 41 wt%, about 42 wt%, about 43 wt%, about 44 wt%, about 45 wt%, about 46 wt%, about 47 wt%, about 48 wt%, about 49 wt%, and about 50 wt%, as well as any range between two of the values above (such as (but not limited to) a range of from about 15 wt% to about 30 wt%, a range of from about 20 wt% to about 25 wt%, etc.).

In certain non-limiting embodiments, the wash reagent may further include other compounds as known in the art or otherwise contemplated herein. For example (but not by way of limitation), the wash reagent may include at least one chelating agent and/or at least one coupling agent. In addition, the wash reagent may further include one or more of any of the compounds listed in Table 1.

The present disclosure is directed to a method according to claim 8 of removing at least one contaminant from a hematology analyzer.

In certain non-limiting embodiments, the hematology analyzer comprises a unified fluid circuit (UFC), and the wash reagent does not substantially damage the UFC.

In certain non-limiting embodiments, the hematology analyzer comprises a vacuum shuttle chamber (VSC), and the at least one contaminant is a buildup in the VSC.

The method may further include one or more additional steps. For example (but not by way of limitation), the method may further include the step of preparing the wash reagent described or otherwise contemplated herein prior to introduction into the hematology analyzer; when this step is present, the non-ionic surfactant (with the alkoxylated fatty alcohol group) is mixed with the other components to form the wash reagent prior to introduction into the hematology analyzer. Alternatively and/or in addition thereto, the method may further include an incubation step (where the wash reagent is incubated within a portion of the hematology analyzer for a certain period of time to enable removal of the buildup). Yet another alternative and/or additional step of the method includes repeating the mixing, insertion, and/or incubation step(s) as needed to substantially remove the contaminant/buildup from the analyzer (and optionally produce fresh wash reagent, if needed).

The present disclosure is directed to a maintenance kit according to claim 9 for a hematology analyzer comprising a unified fluid circuit.

Any of the non-ionic surfactants with an alkoxylated fatty alcohol group described herein above or otherwise contemplated herein may be utilized as the non-ionic surfactant present in the kit. For example (but not by way of limitation), the non-ionic surfactant may include an alkoxylated fatty alcohol group that comprises at least three degrees of ethoxylation, and wherein the number of carbons present in the primary alcohol chain of the alkoxylated fatty alcohol group is in a range of from five to 46 carbons. One particular (but non-limiting) example of a non-ionic surfactant with an alkoxylated fatty alcohol group that can be used in accordance with the present disclosure is NATSURF^{™} 265 (Croda Europe Limited, Snaith, United Kingdom).

In certain non-limiting embodiments, the non-ionic surfactant is present in a volume sufficient to perform about one, about two, about three, about four, about five, about six, about seven, about eight, about nine, or about 10 cleanings, or more, or any range between two of these values (i.e., a range of from about one to about five cleanings, a range of from about one to about three cleanings, etc.). When the kit contains a volume of non-ionic surfactant sufficient to perform more than one cleaning, the combined volume of surfactant may be disposed in a single container, or each volume of the non-ionic surfactant utilized for a single cleaning may be disposed in a separate container. For example, the multiple volumes of non-ionic surfactant may be disposed in individual aliquots (in different containers) that can be utilized for single cleanings.

In a particular (but non-limiting) embodiment, the volume of the non-ionic surfactant present in the maintenance kit is in a range of from about 5 ml to about 100 ml. For example (but not by way of limitation), the volume may be about 5 ml, about 10 ml, about 15 ml, about 20 ml, about 25 ml, about 30 ml, about 35 ml, about 40 ml, about 45 ml, about 50 ml, about 55 ml, about 60 ml, about 65 ml, about 70 ml, about 75 ml, about 80 ml, about 85 ml, about 90 ml, about 95 ml, and about 100 ml, as well as any range between two of these values (i.e., a range of from about 10 ml to about 50 ml, a range of from about 15 ml to about 30 ml, etc.).

In addition to the reagent described in detail herein above, the maintenance kit may further contain other reagent(s) for performing any of the methods described or otherwise contemplated herein. For example (but not by way of limitation), the maintenance kit may further include the wash reagent described herein above but without the non-ionic surfactant added thereto, such that the non-ionic surfactant can be added to the wash reagent at the time of cleaning. In another non-limiting example, the maintenance kit may further include each of the components of the wash reagent described in detail or otherwise contemplated herein. The nature of these additional reagent(s) will depend upon the particular cleaning/maintenance format and the particular analyzer used, and identification thereof is well within the skill of one of ordinary skill in the art; therefore, no further description thereof is deemed necessary. Also, the components/reagents present in the kits may each be in separate containers/compartments, or various components/reagents can be combined in one or more containers/compartments, depending on the cross-reactivity and stability of the components/reagents.

### Examples

Examples are provided hereinbelow. However, the present disclosure is to be understood to not be limited in its application to the specific experimentation, results, and laboratory procedures disclosed herein after. Rather, the Examples are simply provided as one of various embodiments and is meant to be exemplary, not exhaustive.

### Example 1

A clogged UFC unit (due to soils at the Perox VSC and surrounding areas) has always been an issue for hematology systems such as (but not limited to) the ADVIA^{®} 120, ADVIA^{®} 2120, and ADVIA^{®} 2120i Hematology Systems (Siemens Healthcare Diagnostics, Inc., Tarrytown, NY). In the past, a number of reagents and methods have been tested, including the use of caustic reagents such as warm/highly concentrated sodium hydroxide solution as well as organic solvents such as (but not limited to) DMSO, ethanol, isopropanol, and benzol. Though some of these reagents demonstrated the ability to remove the Perox buildups, a sufficiently high concentration level was required for this ability to be seen, and these reagents are not compatible with the UFC material (which is typically acrylic) at such high concentrations.

The currently available wash reagent that is utilized with the ADVIA^{®} Hematology Systems is the EZ wash reagent (reference composition); however, this reagent cannot effectively remove the buildups of the Perox VSC and surrounding pathways. Therefore, various methods of tackling the UFC clogging issue were investigated. FTIR technology was utilized to analyze the buildups, and it was determined that the Perox buildups were the result of some accumulation which initially formed from the precipitation of formaldehyde in Perox 1 and 4-chloro-1-naphthol in Perox 2, with the addition of blood debris. Organic solvents such as (but not limited to) ethanol, isopropanol, benzol, and DMSO were initially proposed to remove the buildups. However, the sufficiently high concentration of the solvents that was required to be effective at removing the buildups was not compatible with the UFC material (which is typically acrylic).

Thus, the formulations of cleaning reagents utilized with other commercially available hematology analyzers were investigated, as shown in Table 1 below. In general, simple sodium hydroxide solutions and enzymatic solutions with antimicrobial agents are the most popular cleaning reagents used by other analyzers. In particular, Beckman Coulter, Inc. uses ethoxylated nonylphenol (i.e., one type of non-ionic surfactant) in their enzymatic cleaning system to enhance its cleaning ability.

**Table 1: Formulations of Wash Reagents Used with Other Commercially Available Hematology Analyzers (reference compositions)**

| Product Name | Enzymatic | Bleach | Antimicrobial | Surfactant | Others |
|---|---|---|---|---|---|
| Abbott* - Cell DYN enzymatic cleaner concentrate | Subtilisin | | 1,3,5-tris(2-hydroxyethyl)hexahydro-1,3,5,-triazine | | |
| Abbott - Cell DYN emerald cleaner | | | Diazolidinyl Urea | ProClin 150 | |
| Horiba** - ABX cleaner | Proteolytic | | | | Organic buffer |
| Horiba - ABX Minoclair | | 13% NaOCl | | | |
| Coulter*** - Clenz cleaning agent | Subtilisin | | Diazolidinyl Urea | ProClin 150 | |
| Coulter - DXH Cleaner | Subtilisin | | | ProClin 150, ethoxylated nonylphenol | |
| Coulter - LH Cleaner | | | | | |
| Diatron⁺ - Hypoclean | | <9% NaOCl | | | |
| Erma⁺⁺ - Cleaning | | | | ProClin | |
| Erma - Emergency Cleaning | | 0.5% NaOCl | | | |
| Mindray⁺⁺⁺ - CBC-3D | | | Sodium azide | | Cycloheximide |
| Mindray - CBC-CAL Plus | | | Sodium azide | | Cycloheximide, potassium hydroxide |
| Mindray - Neo Probe Cleaner | | | ≤10% sodium hypochlorite | | <5% sodium hydroxide |
| Mindray - Neo-CE-Clean | ≤5% Proteolytic | | | | |
| Sysmex⁺⁺⁺⁺ - Cellclean Auto | | anto | | | |

| | | | | | |
|---|---|---|---|---|---|
| *Abbot Laboratories, Chicago, IL; **Horiba, Ltd., Kyoto, Japan; ***Beckman Coulter, Inc., Brea, CA; ⁺Diatron MI Zrt.,Budapest, Hungary; ⁺⁺Erma Inc., Tokyo, Japan; ⁺⁺⁺Mindray Medical International Ltd., Shenzhen, China; ⁺⁺⁺⁺Sysmex Corp., Kobe, Japan. All information obtained from product SDS's. | | | | | |

Cleaning reagents used by medical surgical equipment were also investigated. Like hematology analyzers, the surgical equipment was also found to be contaminated from various sources including (but not limited to) blood stain from hemoglobin and fat from body fluids, as well as salts and other chemicals that are involved in the chemical reaction and detection. International Patent Application Publication No. WO 00/18858 discloses cleaning compositions for the removal of body fluids from surgical instruments and other surface such as operating tables and instrument trays. These cleaning compositions include non-ionic or amphoteric surfactants.

In addition, ethoxylated alcohols or alkoxylated alcohols have been proposed to remove food grease and for aluminum surface cleaning (International Patent Application Publication No. WO 96/26796 and European Patent Application No. 0691421, respectively).

The current reagent utilized with ADVIA^{®} Hematology Systems is the EZ Wash reagent, which contains sodium hydroxide, lauryl dimethyl amine oxide, 2-(2-Ethoxyethoxy)Ethanol, and processed water. The sodium hydroxide functions to keep the solution in an alkaline status (such as (but not limited to) about 12.7). Lauryl dimethyl amine oxide is a non-ionic surfactant that also functions as an antimicrobial agent and may also function to solubilize protein. 2-(2-Ethoxyethoxy)Ethanol functions as a coupling agent and a cleaner. However, this current reagent is not capable of removing all of the buildups on the Perox VSC and surrounding pathways.

These Examples investigated the addition of another non-ionic surfactant, an ethoxylated fatty alcohol, to the EZ Wash reagent, to determine if the ethoxylated fatty alcohol would increase the reagent's cleaning ability. As shown in Examples 2 and 3, the EZ Wash reagent containing the ethoxylated fatty alcohol possessed superior cleaning ability in removing buildups in the Perox VSC and the surrounding pathways compared to the EZ Wash reagent that did not contain the ethoxylated fatty alcohol.

One non-limiting example of ethoxylated fatty alcohol utilized in accordance with the present disclosure is NATSURF^{™} 265 (Croda Europe Limited, Snaith, United Kingdom). NATSURF^{™} 265 has previously been proposed to: (1) remove stubborn UV inks from printing press components (International Patent Application Publication No. WO 2012/054465); (2) be used as a virus inactivation and environmentally friendly detergent in the pharmaceutical industry (Canadian Patent Application No. 2,930,687); and (3) be used as a wetting agent (in combination with other agents that act as acidic components and corrosion inhibitors) in a composition for dissolving and/or inhibiting deposition of scale on a surface of a system such as a heat transfer system (US Patent Application No. US 2013/0137622). However, NATSURF^{™} 265 has not been disclosed for use in reagents for analyzers, such as (but not limited to) diagnostic analyzers, and in particular, hematology analyzers.

It is known that 2-(2-Ethoxyethoxy)Ethanol, an existing chemical component of the currently available EZ Wash reagent, has has two degrees of ethoxylation on ethanol but is not capable of cleaning the buildups in the Perox VSC and the surrounding pathways. Thus, the chemical structure of NATSURF^{™} 265 has at least three degrees of ethoxylation for the cleaning ability to be present. In addition, the number of carbons present in the primary alcohol chain of NATSURF^{™} 265 is in a range of from five to 46 carbons.

As stated herein above, ethoxylated nonylphenol is used in the Beckman Coulter enzymatic cleaning solutions to enhance their cleaning ability (see Table 1). Like NATSURF^{®} 265, ethoxylated nonylphenol is also a non-ionic surfactant. However, ethoxylated nonylphenol is produced by the ethoxylation of alkylphenols. In contrast, ethoxylated fatty alcohols such as (but not limited to) NATSURF^{®} 265 are produced by the ethoxylation of fatty alcohol. From a chemical structure aspect, alkylphenols contain an aromatic ring, while fatty alcohols are usually high molecular weight, straight-chain primary alcohols with the range of carbons from as few as 4-6 to as many as 22-26. Moreover, while NATSURF^{™} 265 is a so called "green detergent," ethoxylated nonylphenol used in the prior art has a mild to medium estrogenic function. As such, this class of detergents has been restricted for commercial "down the drain" applications in Europe and is no longer used by US laundry manufacturers.

As shown in Examples 2-3, the mixture of NATSURF^{™} 265 and EZ Wash reagent effectively removed the buildups in the Perox VSC and surrounding pathways, while not causing any damage to the UFC unit.

The non-ionic surfactant NATSURF^{™} 265 does not present any charge in the aqueous solution, so it is stable and did not interact with the EZ Wash reagent when the two were mixed at room temperature. In the alkaline media (EZ Wash reagent), the cleaning power of NATSURF^{™} 265 was enhanced. In addition, the addition of this non-ionic surfactant to the EZ Wash reagent will extend the use life of the UFC of the hematology analyzer by at least about 1.5 to about 2 years, which is an increase in use life of at least about 30%. This increase in use life will provide a substantial instrument savings (i.e., millions of dollars a year) as well as substantially reduce the amount of field service time associated with an instrument.

### Example 2

The purpose of this Example was to study the use of the non-ionic surfactant NATSURF^{™} 265 (Product Code ET40457, Croda Europe Limited, Snaith, United Kingdom) to remove dried UFC VSC Perox build-up in the ADVIA^{®} 2120i Hematology System (Siemens Healthcare Diagnostics, Inc., Tarrytown, NY).

The ADVIA^{®} 2120i instrument's UFC block is made up of eight acrylic plates. Machined within these plates are the pathways for the fluids and air flow, valves, and four reaction chambers. The Perox reaction chamber is mounted on the outside of the UFC block. The reaction chambers are machined pockets in the UFC where sample and reagents are mixed and the cytochemical reaction takes place.

The Perox vacuum shuttle chamber (VSC) and the immediate surrounding pathways are not effectively cleaned on all ADVIA^{®} 2120i instruments using the maintenance instructions provided in the Operator's guide (10708774, Rev A). During the feasibility test with a dirty UFC block that was freshly removed from a customer's instrument, it was found that the cleaning procedure described in DX008879 Rev 02 provides a more efficient alternative cleaning for the Perox VSC area, and NATSURF^{™} 265 (alkoxylated fatty alcohol), a non-ionic surfactant, was discovered to have a good cleaning ability for the Perox VSC and surrounding pathways after mixing with EZ wash reagent (SMN 10285021). There was no immediate visible damage to the UFC and dome vales after the completion of the cleaning.

Because securing a reliable supply of fresh dirty UFC blocks from a customer's instrument is difficult, dried dirty UFC blocks from Dublin were used in this study to evaluate if NATSURF^{™} 265 can remove the dried build-up from the Perox VSC and surrounding pathways.

Materials: EZ Wash Reagent (SMN 10285021); 1x18 cm Tygon tubing (PN 116-0536-15); ADVIA^{®} 2120i instrument Quality controls (SMN 10316217, 10330063, 10318905); 2x100 ml beaker or large test tube; NATSURF^{™} 265 (product code: ET40457); dried dirty UFC blocks.

### Results:

Ten dried dirty UFC blocks from Dublin were sorted and ranked from #1 to #10, with #1 representing the least amount of Perox build-up and #10 representing an extreme amount of Perox build-up. UFC blocks #1, #5 and #10 were then selected and installed onto the instrument. A cleaning solution containing 20% NATSURF^{™} 265 (1 part NATSURF^{™} 265, 4 parts EZ Wash) was aspirated into the instrument following procedure DX009665.

For UFC block #1 (FIGS. 1A-1D), there was a thin layer of build-up left in Perox VSC chamber after 350 ml of cleaning solution was aspirated (FIG. 1C). However, this thin layer of build-up was quickly removed when the DI-water was aspirated into the Perox VSC chamber (See FIG. 1D). This suggests that alternating cleaning solution and Di-water in the cleaning process can accelerate the cleaning and shorten the required cleaning time.

For UFC block #5, a leaking dome valve was observed after the UFC block was installed onto the instrument. Nevertheless, the cleaning procedure was still performed. 90% build-up was removed after 150 ml of cleaning solution was aspirated, followed by aspiration of 30 ml DI-water into the Perox VSC area (See FIGS. 2A-2D). However, the leakage of the dome valve got worse, and the study was forced to terminate in order to prevent the water entering the instrument compressor.

The build-up on UFC block #10 was so severe that no fluid was able to aspirate into the instrument. Therefore, UFC block #9 was installed and tested (FIG. 3A). Partial blockages were observed with UFC block #9 after installation. A small amount of the cleaning solution was delivered to the Perox VSC area. It took approximately one hour to remove these blockages. The build-up at the Perox VSC chamber and surrounding pathways was removed after 800 ml of cleaning solution and almost 4 hours of cleaning. However, there was still a significant amount of build-up in the Perox VSC chamber (FIG. 3B). To clean the remaining Perox build up, cleaning solution was filled into the Perox VSC chamber at the end of the day and left overnight. The next morning, the majority of build-up was removed from the wall of the Perox VSC chamber (FIG. 3C), and large pieces of build-up were floating inside of the chamber. It took almost another 4 hours and 700 ml of cleaning solution to break down these large pieces of build-up into smaller pieces that could be flushed away from the Perox VSC chamber (FIG. 3D).

In conclusion, the non-ionic surfactant NATSURF^{™} 265 has demonstrated the ability to remove build-up from the Perox VSC chamber and surrounding pathways of a hematology analyzer. It should be noted that cleaning the dried Perox build-up in UFC blocks is harder than cleaning the UFC on instruments with freshly generated build-up, and as such, requires extra time and cleaning solution. Alternating cleaning solution and DI-water can accelerate the cleaning process and shorten the required cleaning time.

While this Example is described in the context of the ADVIA^{®} hematology system, it will be understood that the wash reagents disclosed herein are not limited to use with only these particular systems. Rather, the scope of the present disclosure includes the use of the wash reagents disclosed or otherwise contemplated herein with any hematology analyzers known in the art for which removal of buildups is desired, including (but not limited to) the UniCel^{®} DxH^{™} 800 Coulter Cellular Analysis system and the Coulter LH 500, LH 750, and LH780 hematology analyzers (Beckman Coulter, Inc., Brea, CA).

### Example 3

Based on the ability of the non-ionic surfactant NATSURF^{™} 265 to remove the buildups from the Perox VSC, as demonstrated in Example 2, the new cleaning procedure for the ADVIA^{®} 2102i UFC Perox VSC was verified to confirm that design controls associated with the new cleaning procedure were effective and that the new reagent was compatible with the system.

For example, it was determined whether or not the reagent design was compatible with the UFC dome valve part (067-1282-02) to prevent incompatibility that would cause damage to valves as a result of reagent exposure during the cleaning process. The compatibility testing plan was to complete 28 cleaning events and to confirm that the UFC was not damaged by confirming that the performance of the UFC was acceptable. The data for performance after each cleaning event is shown in Table 2. The performance after each cleaning event passed all specifications and confirmed that the reagent was compatible with the system.

**Table 2: UFC Compatibility Testing Results**

| **Task** | **WBCP (×10³)*** | **Background** | **QC** | **Precision** | **Visual Inspection** |
|---|---|---|---|---|---|
| 1^{st} cleaning | 0.02 | Pass | Pass | Pass | Pass |
| 2^{nd} cleaning | 0.06 | Pass | Pass | Pass | Pass |
| 3^{rd} cleaning | 0.04 | Pass | Pass | Pass | Pass |
| 4^{th} cleaning | 0.06 | Pass | Pass | Pass | Pass |
| 5^{th} cleaning | 0.09 | Pass | Pass | Pass | Pass |
| 6^{th} cleaning | 0.02 | Pass | Pass | Pass | Pass |
| 7^{th} cleaning | 0.02 | Pass | Pass | Pass | Pass |
| 8^{th} cleaning | 0.02 | Pass | Pass | Pass | Pass |
| 9^{th} cleaning | 0.07 | Pass | Pass | Pass | Pass |
| 10^{th} cleaning | 0.04 | Pass | Pass | Pass | Pass |
| 11^{th} cleaning | 0.02 | Pass | Pass | Pass | Pass |
| 12^{th} cleaning | 0.02 | Pass | Pass | Pass | Pass |
| 13^{th} cleaning | 0.01 | Pass | Pass | Pass | Pass |
| 14^{th} cleaning | 0.06 | Pass | Pass | Pass | Pass |
| 15^{th} cleaning | 0.03 | Pass | Pass | Pass | Pass |
| 16^{th} cleaning | 0.07 | Pass | Pass | Pass | Pass |
| 17^{th} cleaning | 0.05 | Pass | Pass | Pass | Pass |
| 18^{th} cleaning | 0.04 | Pass | Pass | Pass | Pass |
| 19^{th} cleaning | 0.03 | Pass | Pass | Pass | Pass |
| 20^{th} cleaning | 0.01 | Pass | Pass | Pass | Pass |
| 21^{st} cleaning | 0.03 | Pass | Pass | Pass | Pass |
| 22^{nd} cleaning | 0.02 | Pass | Pass | Pass | Pass |
| 23^{rd} cleaning | 0.08 | Pass | Pass | Pass | Pass |
| 24^{th} cleaning | 0.01 | Pass | Pass | Pass | Pass |
| 25^{th} cleaning | 0.06 | Pass | Pass | Pass | Pass |
| 26^{th} cleaning | 0.03 | Pass | Pass | Pass | Pass |
| 27^{th} cleaning | 0.04 | Pass | Pass | Pass | Pass |
| 28^{th} cleaning | 0.03 | Pass | Pass | Pass | Pass |

| | | | | | |
|---|---|---|---|---|---|
| Pass/Fall Criteria: WBCP: <0.1 × 10³ cells/L Background: Green lights for background check of Pit, WBCB, and HGB Visual Inspection: Visual check for cracking and leaking | | | | | |

In conclusion, this Example demonstrates that the use of the wash reagent of the present disclosure in an ADVIA^{®} 2120i UFC Perox VSC Cleaning Procedure for the ADVIA^{®} 2120i Hematology System meets with all acceptance criteria and verifies that the new cleaning procedure was effective and that the new reagent was compatible with the system.

## Claims

1. A wash reagent, comprising:
sodium hydroxide in an amount sufficient to provide the wash reagent with an alkaline pH;
lauryl dimethyl amine oxide;
2-(2-ethoxyethoxy)ethanol;
at least one non-ionic surfactant with an alkoxylated fatty alcohol group; and processed water.

2. The wash reagent of claim 1, wherein the alkoxylated fatty alcohol group comprises at least three degrees of ethoxylation.

3. The wash reagent of claim 1 or 2, wherein the number of carbons present in the primary alcohol chain of the alkoxylated fatty alcohol group is in a range of from five to 46 carbons.

4. The wash reagent of any one of claims 1 to 3, wherein sodium hydroxide is present in an amount sufficient to provide the wash reagent with a pH in a range of from 10 to 14, wherein the wash reagent is preferably provided with a pH of 12.7.

5. The wash reagent of any one of claims 1 to 4, wherein the combination of sodium hydroxide, lauryl dimethyl amine oxide, 2-(2-ethoxyethoxy)ethanol, and processed water is present at a combined concentration in a range of from 50 wt% to 95 wt%.

6. The wash reagent of any one of claims 1 to 5, wherein the at least one non-ionic surfactant with the alkoxylated fatty alcohol group is present at a concentration in a range of from about 5 wt% to about 50 wt%, preferably at a concentration in a range of from about 15 wt% to about 30 wt%.

7. The wash reagent of any one of claims 1 to 6, further comprising at least one compound selected from the group consisting of a chelating agent and a coupling agent.

8. A method of removing at least one contaminant from a hematology analyzer, comprising the steps of:
inserting the wash reagent of any one of claims 1 to 7 through at least a portion of the hematology analyzer.

9. The method of claim 8, wherein the hematology analyzer comprises a unified fluid circuit (UFC), and /or wherein the hematology analyzer comprises a vacuum shuttle chamber (VSC), and wherein the at least one contaminant is a buildup in the VSC.

10. A maintenance kit for a hematology analyzer comprising a unified fluid circuit, the kit comprising:
a volume of a non-ionic surfactant with an alkoxylated fatty alcohol group; and
a wash reagent, comprising:
sodium hydroxide in an amount sufficient to provide the wash reagent with an alkaline pH;
lauryl dimethyl amine oxide; and
2-(2-ethoxyethoxy)ethanol; and
processed water;
instructions for diluting the volume of non-ionic surfactant in a wash reagent and passing the wash reagent through at least a portion of the hematology analyzer.

11. The maintenance kit of claim 10, wherein the volume of the non-ionic surfactant is in a range of from 5 ml to 100 ml, preferably 20 ml.

12. The maintenance kit of claim 10 or 11, wherein the alkoxylated fatty alcohol group comprises at least three degrees of ethoxylation.

13. The maintenance kit of any one of claims 10 to 12, wherein the number of carbons present in the primary alcohol chain of the alkoxylated fatty alcohol group is in a range of from five to 46 carbons.

## Patentansprüche

1. Waschreagenz, umfassend:
Natriumhydroxid in einer Menge, die ausreicht, um dem Waschreagenz einen alkalischen pH-Wert zu verleihen;
Lauryldimethylaminoxid;
2-(2-Ethoxyethoxy)ethanol;
mindestens ein nichtionisches Tensid mit einer alkoxylierten Fettalkoholgruppe; und aufgearbeitetes Wasser.

2. Waschreagenz nach Anspruch 1, wobei die alkoxylierte Fettalkoholgruppe mindestens drei Ethoxylierungsgrade umfasst.

3. Waschreagenz nach Anspruch 1 oder 2, wobei die Anzahl an Kohlenstoffen, welche in der primären Alkoholkette der alkoxylierten Fettalkoholgruppe vorliegen, im Bereich von fünf bis 46 Kohlenstoffen liegt.

4. Waschreagenz nach einem beliebigen der Ansprüche 1 bis 3, wobei Natriumhydroxid in einer Menge vorliegt, die ausreicht, um dem Waschreagenz einen pH-Wert im Bereich von 10 bis 14 zu verleihen, wobei dem Waschreagenz vorzugsweise ein pH-Wert von 12,7 verliehen wird.

5. Waschreagenz nach einem beliebigen der Ansprüche 1 bis 4, wobei die Kombination von Natriumhydroxid, Lauryldimethylaminoxid, 2-(2-Ethoxyethoxy)ethanol und aufbereitetem Wasser in einer Konzentration vorliegt, die zusammengenommen im Bereich von 50 Gew.-% bis 95 Gew.-% liegt.

6. Waschreagenz nach einem beliebigen der Ansprüche 1 bis 5, wobei das mindestens eine nichtionische Tensid mit der alkoxylierten Fettalkoholgruppe in einer Konzentration, die im Bereich von ungefähr 5 Gew.-% bis ungefähr 50 Gew.-% liegt, vorzugsweise in einer Konzentration vorliegt, die im Bereich von ungefähr 15 Gew.-% bis ungefähr 30 Gew.-% liegt.

7. Waschreagenz nach einem beliebigen der Ansprüche 1 bis 6, das weiterhin mindestens eine Verbindung umfasst, die aus der Gruppe ausgewählt ist, welche aus einem Chelatbildner und einem Kupplungsmittel besteht.

8. Verfahren zum Entfernen mindestens einer Verunreinigung aus einem hämatologischen Analysegerät, wobei es die folgenden Schritte umfasst:
Einbringen des Waschreagenzes nach einem beliebigen der Ansprüche 1 bis 7 zumindest in einen Teilbereich des hämatologischen Analysegeräts.

9. Verfahren nach Anspruch 8, wobei das hämatologische Analysegerät einen vereinheitlichten Fluidkreislauf (UFC) umfasst und/oder wobei das hämatologische Analysegerät eine Vakuumpendelkammer (VSC) umfasst und wobei die mindestens eine Verunreinigung sich in der VSC angesammelt hat.

10. Wartungskit für ein hämatologisches Gerät, welches einen vereinheitlichten Fluidkreislauf umfasst, wobei das Kit Folgendes umfasst:
ein Volumen an einem nichtionischen Tensid mit einer alkoxylierten Fettalkoholgruppe; und
ein Waschreagenz, das Folgendes umfasst:
Natriumhydroxid in einer Menge, die ausreicht, um dem Waschreagenz einen alkalischen pH-Wert zu verleihen;
Lauryldimethylaminoxid; und
2-(2-Ethoxyethoxy)ethanol; und
aufgearbeitetes Wasser;
Anweisungen zum Verdünnen des Volumens an nichtionischem Tensid in einem Waschreagenz und Leiten des Waschreagenzes zumindest durch einen Teilabschnitt des hämatologischen Analysegeräts.

11. Wartungskit nach Anspruch 10, wobei das Volumen des nichtionischen Tensids im Bereich von 5 mL bis 100 mL liegt, wobei es vorzugsweise 20 mL beträgt.

12. Wartungskit nach Anspruch 10 oder 11, wobei die alkoxylierte Fettalkoholgruppe mindestens drei Ethoxylierungsgrade umfasst.

13. Wartungskit nach einem beliebigen der Ansprüche 10 bis 12, wobei die Anzahl an Kohlenstoffen, welche in der primären Alkoholkette der alkoxylierten Fettalkoholgruppe vorliegen, im Bereich von fünf bis 46 Kohlenstoffen liegt.

## Revendications

1. Réactif de lavage, comprenant :
de l'hydroxyde de sodium en une quantité suffisante pour donner un pH alcalin au réactif de lavage ;
de l'oxyde de lauryldiméthylamine ;
du 2-(2-éthoxyéthoxy)éthanol ;
au moins un agent tensioactif non-ionique ayant un groupe d'alcool gras alcoxylé ; et de l'eau traitée.

2. Réactif de lavage suivant la revendication 1, dans lequel le groupe alcool gras alcoxylé comprend au moins trois degrés d'éthoxylation.

3. Réactif de lavage suivant la revendication 1 ou 2, dans lequel le nombre de carbones présent dans la chaîne alcool primaire du groupe alcool gras alcoxylé est dans la plage de 5 et 46 carbones.

4. Réactif de lavage suivant l'une quelconque des revendications 1 à 3, dans lequel l'hydroxyde de sodium est présent en une quantité suffisante pour donner au réactif de lavage un pH dans la plage de 10 à 14, le réactif de lavage ayant, de préférence un pH de 12,7.

5. Réactif de lavage suivant l'une quelconque des revendications 1 à 4, dans lequel la combinaison d'hydroxyde de sodium, d'oxyde de lauryldiméthylamine, de 2(2-éthoxyéthoxy)éthanol et d'eau traitée est présente en une concentration combinée dans une plage de 50 % en poids à 95 % en poids.

6. Réactif de lavage suivant l'une quelconque des revendications 1 à 5, dans lequel le au moins un agent tensioactif non-ionique ayant le groupe alcool gras alcoxylé est présent en une concentration dans une plage d'environ 5 % en poids à environ 50 % en poids, de préférence en une concentration dans une plage d'environ 15 % en poids à environ 30 % en poids.

7. Réactif de lavage suivant l'une quelconque des revendications 1 à 6, comprenant en outre un composé choisi dans le groupe consistant en un agent de chélation et un agent de couplage.

8. Procédé d'élimination d'au moins un polluant d'un analyseur d'hématologie, comprenant les stades dans lesquels :
on insère le réactif de lavage suivant l'une quelconque des revendications 1 à 7, dans au moins une partie de l'analyseur d'hématologie.

9. Procédé suivant la revendication 8, dans lequel l'analyseur d'hématologie comprend un circuit de fluide unifié (UFC), et/ou dans lequel l'analyseur d'hématologie comprend une chambre shuttle sous vide (VSC), et dans lequel le au moins un polluant est une accumulation dans la VSC.

10. Trousse d'entretien d'un analyseur d'hématologie comprenant un circuit fluide unifié, la trousse comprenant :
un volume d'un agent tensioactif non ionique ayant un groupe alcool gras alcoxylé ; et un réactif de lavage comprenant :
de l'hydroxyde de sodium en une quantité suffisante pour donner un pH alcalin au réactif de lavage ;
de l'oxyde de lauryldiméthylamine ;
du 2-(2-éthoxyéthoxy)éthanol ; et
de l'eau traitée ;
des instructions pour diluer le volume de l'agent tensioactif non ionique dans un réactif de lavage et faire passer le réactif de lavage dans au moins une partie de l'analyseur d'hématologie.

11. Trousse d'entretien suivant la revendication 10, dans lequel le volume de l'agent tensioactif non ionique est dans une plage de 5 ml à 100 ml, de préférence de 20 ml.

12. Trousse d'entretien suivant la revendication 10 ou 11, dans lequel le groupe alcool gras alcoxylé comprend au moins trois degrés d'éthoxylation.

13. Trousse d'entretien suivant l'une quelconque des revendications 10 à 12, dans lequel le nombre de carbones présent dans la chaîne alcool primaire du groupe alcool gras alcoxylé est dans une plage de cinq à 46 carbones.
